# EUROPEAN PATENT SPECIFICATION

(11) **EP 2 690 428 B1**
(45) Date of publication and mention of the grant of the patent: **13.11.2019**
(21) Application number: 12760097.1
(22) Date of filing: 01.03.2012
(51) Int. Cl.: A61B 5/145, G01N 21/84, G01N 21/78

(54) **COMPONENT MEASUREMENT DEVICE**
KOMPONENTENMESSVORRICHTUNG
DISPOSITIF DE MESURE DE COMPOSANT

(30) Priority: 23.03.2011 JP 2011063686
(43) Date of publication of application: 29.01.2014
(73) Proprietor: Terumo Kabushiki Kaisha, Tokyo 151-0072 (JP)
(72) Inventor: MORITA Takashi, Ashigarakami-gun, Kanagawa 259-0151 (JP); NAGASAWA Yasushi, Ashigarakami-gun, Kanagawa 259-0151 (JP); MURAYAMA Masami, Shinagawa-ku, Tokyo 141-0022 (JP); NAGAOKA Yoshio, Shinagawa-ku, Tokyo 141-0022 (JP); IZUMI Eiki, Shinagawa-ku, Tokyo 141-0022 (JP)
(74) Representative: TBK
(86) International application number: PCT/JP2012/055216
(87) International publication number: WO 2012/128014

(56) References cited:
- WO-A2-2012/042023
- JP-A- 11 276 498
- JP-A- 2000 509 498
- JP-A- 2005 091 315
- JP-A- 2006 098 227
- JP-A- 2006 507 494
- JP-A- 2010 281 751
- US-A1- 2005 095 697
- US-A1- 2006 098 203
- US-B2- 7 847 946

## Description

### Technical Field

The present invention relates to a component measuring device for optically measuring a biological component in a body fluid, for example.

### Background Art

A component measuring device capable of optical measurement is used when detecting a biological component in a body fluid such as blood, urine, or the like, and measuring the component quantity, quality, or the like of the biological component.

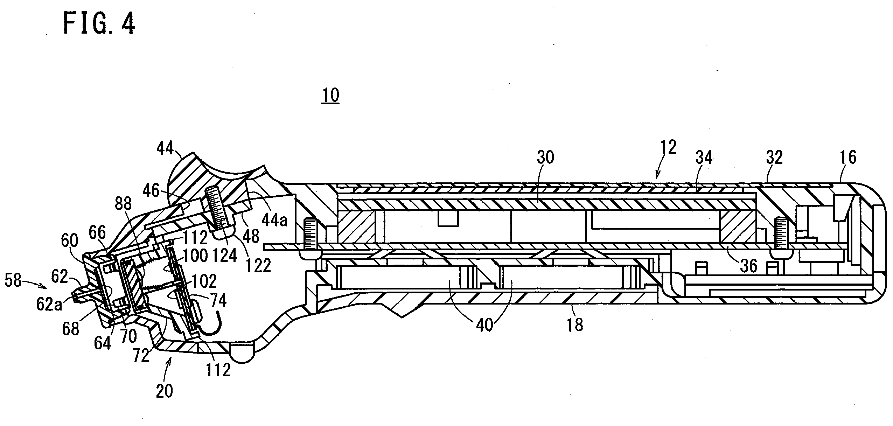

For example, JP-T-2008-544265 discloses an analytic system in which a transmission region formed of a light transmitting synthetic resin is disposed in an optical module base (hereinafter referred to as a block body). This analytic system measures a biological component by emitting light for photometric analysis (hereinafter referred to as irradiation light) from a light source, irradiating a test field via the transmission region, and making the reflected light incident on a detector (hereinafter referred to as a light receiving element) via the same transmission region (see FIG. 4 of JP-T-2008-544265). A lens for condensing the irradiation light at a predetermined position of the test field (which lens will hereinafter be referred to as an irradiation light lens) is provided in this transmission region.

In addition, though not described in JP-T-2008-544265, a reflected light lens may be provided on an optical path (transmission region, for example) of the reflected light. When the reflected light lens condenses the reflected light onto the light receiving element, the accuracy of detection of the reflected light by the light receiving element can be improved. US2005/095697 also shows a device for optically measuring a biological sample using light emitting and light receiving elements with a sensor to receive reflected light from the sample via an aspheric lens.

### Summary of Invention

In the component measuring device of the structure including the reflected light lens in the block body, it is difficult to make a light shielding property of the optical path of the reflected light in the block body and the mountability of the reflected light lens compatible with each other. For example, in the analytic system of JP-T-2008-544265, the light shielding property of the optical path of the reflected light is secured by making an opening on reflected light incidence side of the block body as narrow as possible. In this case, however, it is difficult to mount a transmission region (assumed to correspond to the reflected light lens) of a larger shape than the opening in the block body. That is, when importance is attached to the light shielding property of the optical path of the reflected light, the mountability of the lens in the block body is degraded, and efficiency of work in a device assembling process is decreased.

On the other hand, when importance is attached to the mountability of the reflected light lens, a structure is suitable in which a mounting portion opened so as to communicate with the optical path of the reflected light is formed in the block body and the reflected light lens is inserted into the mounting portion from one direction. In this structure, however, the mounting portion needs to be formed in a small size so as to secure the light shielding property of the optical path of the reflected light, and the shape of the reflected light lens is limited. Therefore, when the reflected light lens is disposed on the optical path of the reflected light, aberration (spherical aberration in particular) is caused in the reflected light after passing through the reflected light lens. As a result, the accuracy of detection of the light receiving element receiving the reflected light is not improved.

The present invention has been made in view of the above problems. It is an object of the present invention to provide a component measuring device with an increased device assembly efficiency and an improved measurement accuracy.

In order to achieve the above object, according to the present invention, there is provided a component measuring device for irradiating a measurement object with irradiation light for measurement from a light emitting element, detecting reflected light reflected from the measurement object in a light receiving element, and measuring a component in a body fluid impregnated into the measurement object on a basis of a detected value of the reflected light, the component measuring device being characterized by including: a block body having a mounting portion having an opening so as to be opposed to the measurement object, and having a reflected light optical path for guiding the reflected light to the light receiving element, the reflected light optical path communicating with the mounting portion; and a reflected light lens for condensing the reflected light onto the light receiving element, wherein the reflected light lens is formed as an aspheric lens for reducing at least spherical aberration of the reflected light on a basis of a position of disposition of the light receiving element, and is disposed on the reflected light optical path by being inserted into the mounting portion from the opening.

According to the above, the reflected light lens is inserted into the mounting portion from the opening of the block body. Thus, the reflected light lens can be easily mounted in the block body. This increases the efficiency of device assembly. In addition, the reflected light lens is formed as an aspheric lens. Thus, the reflected light whose spherical aberration is greatly reduced can be condensed on the light receiving element. As a result, the component measuring device can detect the reflected light accurately, and improve the accuracy of measurement of a biological component. The component measuring device according to the present invention thus combines an increased efficiency of device assembly by a structure allowing a reflected light lens to be mounted easily with an improved accuracy of measurement of a biological component by greatly reducing the spherical aberration of reflected light due to the reflected light lens.

In addition, the block body has an irradiation light optical path for guiding the irradiation light to the measurement object, an irradiation light lens for condensing the irradiation light onto the measurement object may be disposed on the irradiation light optical path, and the reflected light lens and the irradiation light lens may be arranged so as to share a retained portion retained by the mounting portion, and molded integrally with each other.

Even when the reflected light lens and the irradiation light lens are thus molded integrally with each other as one lens, the lens can be mounted in the block body easily. In addition, the number of parts is reduced by molding the reflected light lens and the irradiation light lens integrally with each other. It is therefore possible to reduce manufacturing cost, and further increase the efficiency of device assembly.

In this case, an optical axis of the irradiation light lens and an optical axis of the reflected light lens are formed substantially in parallel with each other, and the aspheric lens may be formed in a shape reducing the spherical aberration of the reflected light incident at a predetermined angle with the optical axis of the reflected light lens.

When the optical axis of the irradiation light lens and the optical axis of the reflected light lens are thus formed substantially in parallel with each other, processing when the irradiation light lens and the reflected light lens are molded integrally with each other can be performed easily.

Further, preferably, the reflected light lens includes a projecting portion projecting by a predetermined amount from the retained portion toward the reflected light optical path, and a condensing portion formed as the aspheric lens on the projecting portion.

When the reflected light lens thus has the projecting portion projecting by a predetermined amount from the retained portion, the condensing portion can be securely positioned at a predetermined position in the reflected light optical path. Hence, even when an external force or the like is applied to the component measuring device, positional displacement of the reflected light lens can be avoided. Thus, the reflected light can be stably condensed on the light receiving element.

An opening end portion of the mounting portion can be swaged to an inside in a state of the reflected light lens being inserted within the mounting portion.

When the opening end portion of the mounting portion is swaged to the inside, the reflected light lens can be prevented from falling off.

In addition, the component measuring device is suitable for use as a blood glucose level measuring device for measuring mainly a blood glucose level of blood components.

According to the present invention, the efficiency of a device assembling process is increased by the structure allowing the reflected light lens to be mounted easily, and the accuracy of measurement of a biological component can be improved by greatly reducing the spherical aberration of the reflected light due to the reflected light lens.

### Brief Description of Drawings

[FIG. 1]

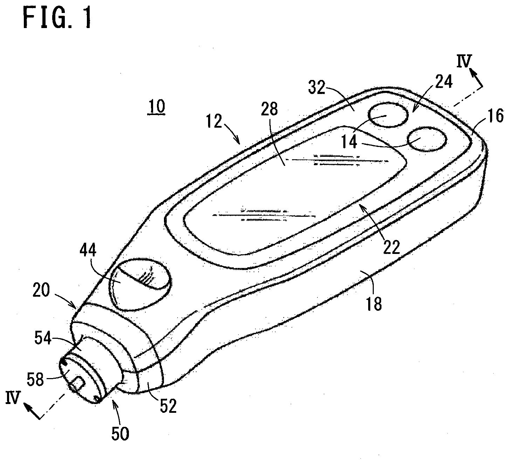

   FIG. 1 is a perspective view of a general constitution of a blood glucose level measuring device according to an embodiment of the present invention.
[FIG. 2]

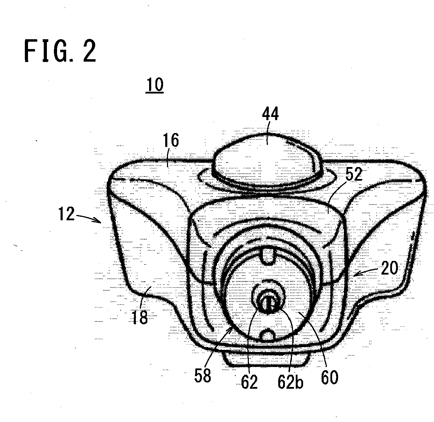

   FIG. 2 is a front view of the blood glucose level measuring device in FIG. 1.
[FIG. 3]

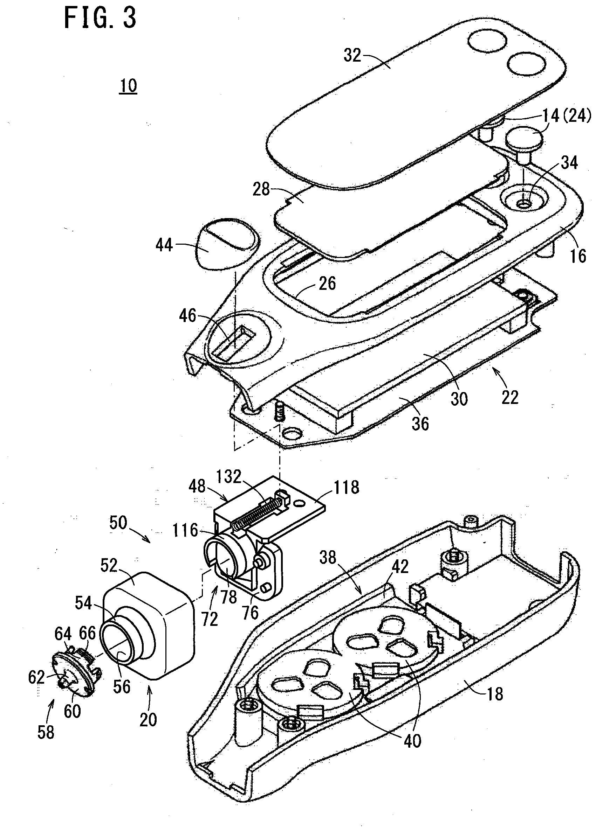

   FIG. 3 is an exploded perspective view of the blood glucose level measuring device in FIG. 1.
[FIG. 4]
   FIG. 4 is a sectional view of the blood glucose level measuring device, the sectional view being taken along a line IV-IV of FIG. 1.
[FIG. 5]

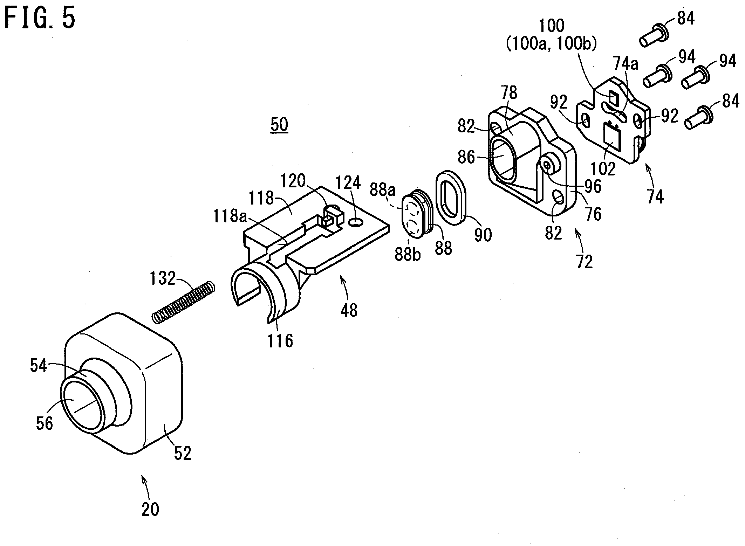

   FIG. 5 is an exploded perspective view of a measuring portion of the blood glucose level measuring device in FIG. 1.
[FIG. 6]

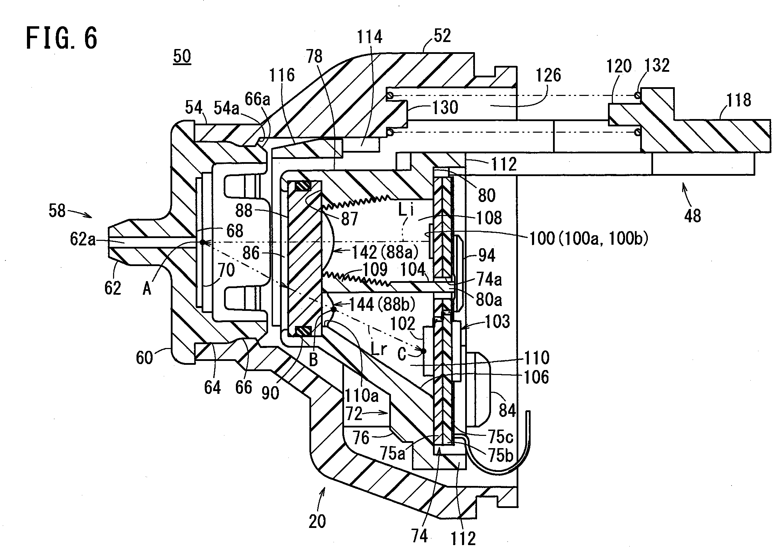

   FIG. 6 is a side sectional view showing the measuring portion of the blood glucose level measuring device in FIG. 4 in enlarged dimension.
[FIG. 7]

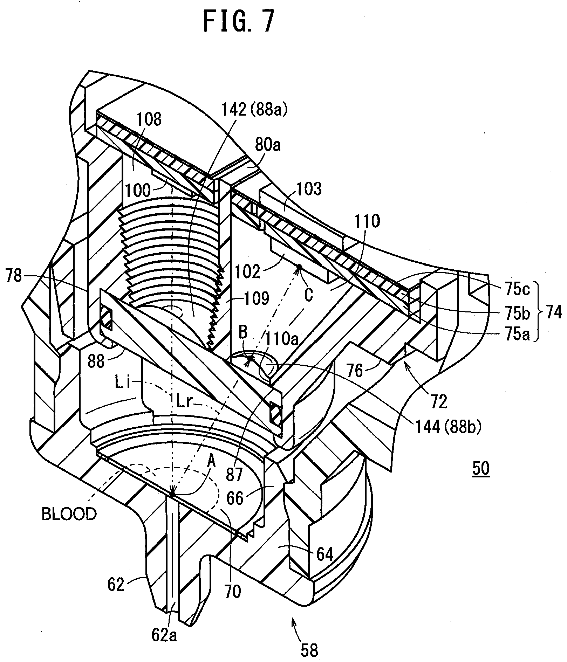

   FIG. 7 is a diagram of assistance in explaining a case where a blood component is detected in the measuring portion of the blood glucose level measuring device in FIG. 1.
[FIG. 8]

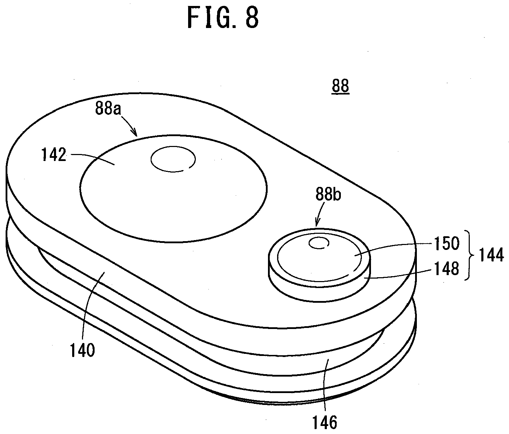

   FIG. 8 is a perspective view of a lens of the blood glucose level measuring device according to the embodiment of the present invention.
[FIG. 9]

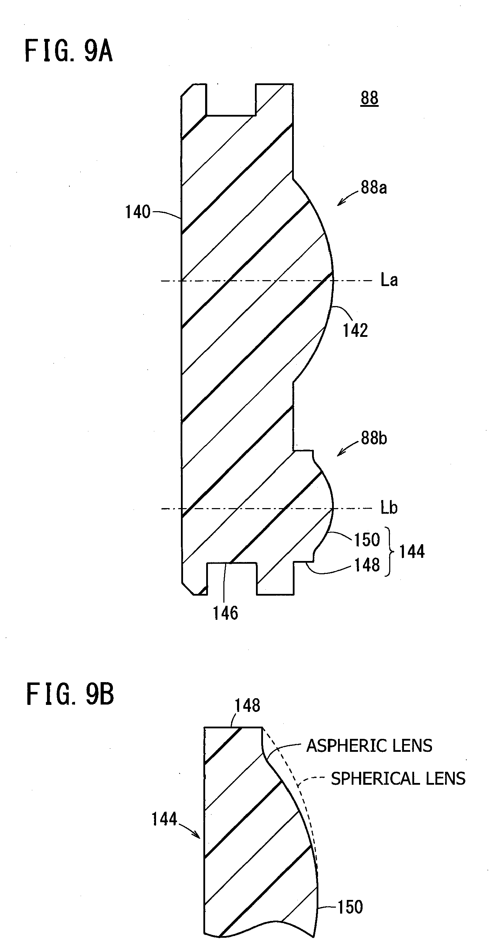

   FIG. 9A is a side sectional view of the lens shown in FIG. 8, and FIG. 9B is a side sectional view showing a part of a reflected light convex portion shown in FIG. 9A in enlarged dimension.
[FIG. 10]

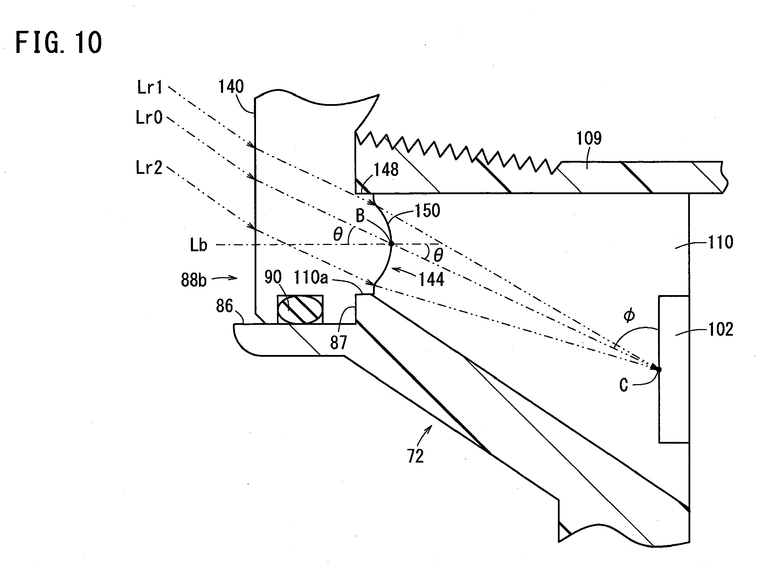

   FIG. 10 is a schematic diagram of assistance in explaining the condensing of reflected light by a reflected light lens shown in FIG. 9.
[FIG. 11]

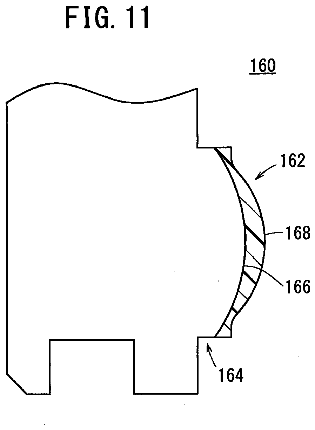

   FIG. 11 is a schematic diagram of assistance in explaining a modification of the reflected light lens.

### Mode for Carrying Out the Invention

A preferred embodiment of a component measuring device according to the present invention will hereinafter be cited and described in detail with reference to the accompanying drawings.

In the description of the present embodiment, a blood glucose level measuring device for measuring mainly a blood glucose level of blood components will be described in detail as a component measuring device. This blood glucose level measuring device measures the blood glucose level of blood collected by a doctor, a nurse, a diabetic, or the like, and manages the blood glucose level measurement data. Incidentally, needless to say, the component measuring device is not limited to the present blood glucose level measuring device.

FIG. 1 is a perspective view of a general constitution of the blood glucose level measuring device (component measuring device) according to the embodiment of the present invention. FIG. 2 is a front view of the same device. FIG. 3 is an exploded perspective view of the same device. FIG. 4 is a side sectional view taken along a line IV-IV of FIG. 1.

As shown in FIG. 1 and FIG. 2, the blood glucose level measuring device 10 has a casing 12 constituting an external appearance. The casing 12 is formed in a three-dimensional shape that is slightly elongate and which fits a hand well so that a person can easily perform pressing operation on operating switches 14 while holding the casing 12 with one hand. The casing 12 includes an upper case 16, a lower case 18, and a distal end case 20. The casing 12 is assembled by vertically superposing the upper case 16 on the lower case 18 and mounting the distal end case 20 on distal end portions of the upper case 16 and the lower case 18. In addition, a display portion 22 for displaying an input item for information necessary to measure a blood glucose level, a checking item, a measurement result, and the like and an operating portion 24 including two operating switches 14 are arranged in the casing 12.

As shown in FIG. 3, the display portion 22 of the blood glucose level measuring device 10 has a liquid crystal cover 28 fitted into an opening window 26 formed in the upper case 16, and includes a liquid crystal panel 30 under the liquid crystal cover 28. Incidentally, a front surface panel 32 formed in an appropriate size for covering the liquid crystal cover 28 and the two operating switches 14 is laminated to an upper surface of the upper case 16.

In the operating portion 24, the two operating switches 14 are inserted into respective insertion holes 34 provided in the upper surface of the upper case 16. The operating portion 24 allows various kinds of operations such as on/off operation of the blood glucose level measuring device 10 and the like to be performed via the operating switches 14.

The liquid crystal panel 30 of the display portion 22 and a main wiring board 36 for controlling the present blood glucose level measuring device 10 are arranged on a back side of the upper case 16 provided with the display portion 22 and the operating portion 24 (within the casing 12). A predetermined wiring circuit is formed on the main wiring board 36 by printing. The main wiring board 36 is mounted with a microcomputer for performing predetermined processing, storage devices such as a ROM storing a predetermined program, a RAM, and the like, and other electronic parts (active elements, passive elements, and the like) (neither is shown).

In addition, a battery housing portion 38 is provided on an upper surface side of the lower case 18 (within the casing 12). A button type battery 40 as a portable power supply is housed in the battery housing portion 38. The battery housing portion 38 is covered in such a manner as to be able to be opened and closed by a battery lid 42 detachably formed in the lower case 18. In the blood glucose level measuring device 10, the control of the main wiring board 36 and the like or the display of the display portion 22 and the like are performed on power from the button type battery 40. Incidentally, the power supply used in the blood glucose level measuring device 10 is not limited to a button type battery, but may be a round type dry battery, a rectangular type dry battery, or a secondary battery, or may be connected to an external power supply via a power cord.

As shown in FIG. 1, the casing 12 having the upper case 16 and the lower case 18 superposed on each other is formed so as to be tapered from a middle portion to a distal end portion and bent to the lower case 18 side as a whole. The distal end case 20 is attached to the distal end portion, and is formed as a casing for a measuring portion 50 for optically detecting blood.

In addition, a long hole 46 for guiding the movement of an ejecting operating element 44 is provided near to the distal end portion in the upper surface of the upper case 16 (see FIG. 3). The long hole 46 extends linearly for a predetermined length in the forward-rearward direction of the casing 12. A leg portion 44a of the ejecting operating element 44 is slidably inserted in the long hole 46 (see FIG. 4). An ejecting member 48 is screwed to the leg portion 44a within the casing 12. A sliding operation of the ejecting member 48 is enabled by the ejecting operating element 44.

As shown in FIG. 3, the distal end case 20 is formed by a prismatic portion 52 attached to the upper case 16 and the lower case 18 and a cylindrical portion 54 formed on a distal end of the prismatic portion 52. Various members for optically measuring blood are attached to the inside of the prismatic portion 52. The cylindrical portion 54 has an opening in a distal end surface thereof. A measuring tip 58 is detachably attached to the opening portion 56.

The measuring tip 58 includes a base portion 60 formed in the shape of a disk, a nozzle 62 formed on a distal end surface side of the base portion 60, and an engaging portion 64 formed on an opposite surface side from the nozzle 62. The base portion 60 is formed such that the outside diameter of the base portion 60 substantially coincides with the outside diameter of the cylindrical portion 54. The nozzle 62 erects from the center of the base portion 60. A collecting hole 62a penetrating from a distal end surface to a back surface is formed on the central axis of the nozzle 62 (see FIG. 4). In addition, a recessed groove 62b for facilitating the absorption of blood is formed in the distal end surface of the nozzle 62 (see FIG. 2).

The engaging portion 64 of the measuring tip 58 is formed in a cylindrical shape, and is formed with such an outside diameter as to be fitted into the opening portion 56 of the cylindrical portion 54. The engaging portion 64 is formed such that four locking pawls (locking portions) 66 having an elastic force project rearward. A projection portion 66a engaged with a ridge 54a formed within the cylindrical portion 54 when the locking pawls 66 are inserted into the cylindrical portion 54 is formed on an outer circumference side of each of the locking pawls 66 (see FIG. 6). The projection portion 66a goes over the ridge 54a and is locked to the ridge 54a, whereby the measuring tip 58 is attached to the cylindrical portion 54.

In addition, as shown in FIG. 4, a test paper housing portion 68 communicating with the collecting hole 62a is provided on the inside of the engaging portion 64. A test paper (measurement object) 70 impregnated with blood when the blood is collected is housed in the test paper housing portion 68. The blood glucose level measuring device 10 measures the component of the blood by irradiating the test paper 70 with irradiation light and receiving reflected light from the test paper 70.

FIG. 5 is an exploded perspective view of the measuring portion 50 of the blood glucose level measuring device 10 in FIG. 1. FIG. 6 is an enlarged sectional view of the measuring portion 50. FIG. 7 is a diagram of assistance in explaining the detection of a blood component in the measuring portion 50.

The measuring portion 50 of the blood glucose level measuring device 10 optically detects a component included in the blood collected in the measuring tip 58. As shown in FIG. 5, the measuring portion 50 includes the distal end case 20, a photometric block (block body) 72, a board 74, the ejecting member 48, and the like. As already described, the distal end case 20 includes the prismatic portion 52 and the cylindrical portion 54, and is attached to the distal end portion of the casing 12 having the upper case 16 and the lower case 18 superposed on each other. The distal end case 20 is for example molded of a synthetic resin such as an ASB resin, polycarbonate, or the like.

The photometric block 72 is a member that retains the board 74 detecting the blood component and which is attached to the inside of the distal end case 20. The photometric block 72 can be molded of a same material as the distal end case 20. The photometric block 72 includes a proximal end portion 76 in the shape of a flat plate and a projecting portion 78 projecting from the proximal end portion 76 in a direction of the distal end.

As shown in FIG. 6, the proximal end portion 76 of the photometric block 72 is provided with the projecting portion 78 on a front surface of the proximal end portion 76 and a board disposing portion 80 on a rear surface of the proximal end portion 76. The board disposing portion 80 is formed in a flat shape on which the board 74 can be disposed. A positioning projection 80a for positioning the board 74 erects from substantially the central portion of the board disposing portion 80. The positioning projection 80a penetrates through an engaging hole 74a of the board 74, and is interposed between a light emitting element 100 and a light receiving element 102 to be described later, thereby having a function of preventing direct propagation of light from the light emitting element 100 to the light receiving element 102.

In addition, two opening portions (an irradiation light board side opening portion 104 and a reflected light board side opening portion 106) are formed in the board disposing portion 80. The irradiation light board side opening portion 104 communicates with an irradiation light optical path 108. The reflected light board side opening portion 106 communicates with a reflected light optical path 110. The irradiation light optical path 108 and the reflected light optical path 110 each penetrate the inside of the photometric block 72 (the proximal end portion 76 and the projecting portion 78), and are each shielded from the light of the other by a wall portion 109 continuous with the positioning projection 80a. In the blood glucose level measuring device 10, both of the irradiation light optical path 108 and the reflected light optical path 110 are thus formed in the photometric block 72, whereby the number of parts can be reduced, and manufacturing cost can be reduced.

Incidentally, a partition wall 112 projecting rearward from the board disposing portion 80 is formed on a rear surface of the proximal end portion 76. The partition wall 112 in a state of the board 74 being disposed on the board disposing portion 80 is formed so as to enclose all sides of a rear surface of the photometric block 72 and project further rearward than the board 74, thus having a function of preventing the contact of liquids with the board 74 and the sticking of dust and the like to the board 74. Further, an attaching screw hole 82 is formed at two positions of the proximal end portion 76 (see FIG. 5). The photometric block 72 is attached to the distal end case 20 by inserting attaching screws 84 into the attaching screw holes 82 from the rear and screwing the attaching screws 84 into attaching female screws (not shown) formed in the distal end case 20.

As shown in FIG. 5, the projecting portion 78 of the photometric block 72 is formed as a substantially cylindrical body both side surfaces of which are in a linear shape and whose upper surface and lower surface are in the shape of an arc. A projecting portion side opening portion 86 is formed in a front surface of the projecting portion 78. The projecting portion side opening portion 86 has a mounting portion 87 formed by cutting away an inner surface of the projecting portion side opening portion 86 to a predetermined depth in an axial direction of the projecting portion 78. The irradiation light optical path 108 and the reflected light optical path 110 communicate with the mounting portion 87. The mounting portion 87 retains a lens 88 inserted from a front side.

The lens 88 retained by the photometric block 72 is an integral type having an irradiation light lens 88a formed on an upper side and a reflected light lens 88b formed on a lower side. The lens 88 is mounted in the mounting portion 87 with an O-ring 90 fitted in a side circumferential surface of the lens 88 at a time of mounting, whereby the irradiation light optical path 108 and the reflected light optical path 110 are sealed. A concrete structure and action and effect of the lens 88 will be described later.

The board 74 of the measuring portion 50 is formed in such a shape as to be able to be disposed in the board disposing portion 80. A board side screw hole 92 is drilled in predetermined positions (two positions) of the board 74. The board 74 is disposed in the photometric block 72 by inserting board screws 94 into the board side screw holes 92 from the rear and screwing the board screws 94 into board fixing holes 96 formed in the board disposing portion 80. The engaging hole 74a engaged with the positioning projection 80a is formed in substantially a central portion of the board 74.

As shown in FIG. 6, two light emitting elements 100 (a first light emitting element 100a and a second light emitting element 100b) for emitting irradiation light and the light receiving element 102 for receiving reflected light are mounted on a surface of the board 74 which surface is opposed to the board disposing portion 80. An amplifier (AMP) 103 electrically connected to the light receiving element 102 and other electronic parts (not shown) necessary to detect a blood component are mounted on a surface on an opposite side from the surface on which the light emitting elements 100 and the light receiving element 102 are mounted. Further, the board 74 according to the present embodiment is formed as a laminated structure of a plurality of flat board layers (a first flat board layer 75a and a second flat board layer 75b) and a light shielding layer 75c, and is configured to prevent extraneous light and the like from entering the irradiation light optical path 108 and the reflected light optical path 110.

The first light emitting element 100a and the second light emitting element 100b are provided to emit irradiation light of different wavelengths. Light emitting diodes (LEDs) for emitting light of predetermined wavelengths, for example, can be applied as the light emitting elements 100. In addition, a photodiode (PD), for example, can be applied as the light receiving element 102. Incidentally, in the present embodiment, miniaturization of the board 74 and miniaturization of the blood glucose level measuring device 10 are achieved by mounting the light emitting elements 100 and the light receiving element 102 without a shell-shaped exterior (transmission body) on the board 74.

The amplifier 103 has a function of amplifying a detection signal output by the light receiving element 102. An operational amplifier, for example, can be applied as the amplifier 103.

When the board 74 is disposed in the board disposing portion 80 of the photometric block 72, the light emitting elements 100 and the light receiving element 102 are disposed so as to face the board disposing portion 80. In a state in which the board 74 is disposed in the board disposing portion 80, the light emitting elements 100 are disposed on the proximal end of the irradiation light optical path 108, and the light receiving element 102 is disposed on the proximal end of the reflected light optical path 110. As shown in FIG. 7, the light emitting elements 100 at a time of measurement emit irradiation light Li to irradiate the test paper 70 with the irradiation light through the irradiation light optical path 108 and the irradiation light lens 88a. The light receiving element 102 receives reflected light Lr reflected from the test paper 70 through the reflected light lens 88b and the reflected light optical path 110.

In a state in which the photometric block 72 is attached to the distal end case 20, a clearance 114 is formed between an inner circumferential surface of the distal end case 20 and a side surface of the projecting portion 78 of the photometric block 72. The ejecting member 48 is slidably disposed in the clearance 114.

As shown in FIG. 5, the ejecting member 48 is formed by integrally molding a pushing-out portion 116 formed on the distal end and a sliding plate 118 to which the pushing-out portion 116 is fixed and which is slidable by a predetermined distance.

The pushing-out portion 116 of the ejecting member 48 is formed in the shape of an arc obtained by cutting away a lower portion of a cylindrical shape by a predetermined amount.

The sliding plate 118 is formed in the shape of a flat plate extending from the pushing-out portion 116 to the rear. A central portion of the sliding plate 118 is cut away in a longitudinal direction. A spring projection 120 is formed at a rear end of the cut-away portion 118a. In addition, an ejecting member side screw hole 124 for fixation to the leg portion 44a of the ejecting operating element 44, into which screw hole 124 an ejecting screw 122 (see FIG. 4) is screwed, is drilled in a rear portion of the sliding plate 118.

As shown in FIG. 6, an ejecting member disposing portion 126 for housing the distal end of the ejecting member 48 is formed in the distal end case 20. The ejecting member disposing portion 126 is formed on an upper side within the prismatic portion 52, and is configured to support both side end portions of the sliding plate 118. The ejecting member disposing portion 126 has a spring disposing projection 130 projecting rearward.

As shown in FIG. 5 and FIG. 6, the ejecting member 48 is disposed in the ejecting member disposing portion 126 with a spring member 132 disposed in the cut-away portion 118a. In this case, the spring projection 120 is inserted into one end of the spring member 132, and the spring disposing projection 130 is inserted into another end of the spring member 132.

In a state in which the photometric block 72 and the ejecting member 48 are disposed in the distal end case 20, the pushing-out portion 116 is disposed on a peripheral surface (upper surface and both side surfaces) of the projecting portion 78 of the photometric block 72. In addition, the ejecting member 48 is slidably disposed in the longitudinal direction of the casing 12. When the ejecting member 48 slides, the pushing-out portion 116 moves back and forth on the periphery of the projecting portion 78 (that is, in the clearance 114). When the measuring tip 58 is attached to the distal end case 20, the pushing-out portion 116 pushes out the locking pawls 66 of the measuring tip 58 by the movement of the ejecting member 48 in the direction of the distal end. Thereby the measuring tip 58 can be removed from the casing 12.

Characteristic parts of the lens 88 according to the present embodiment will next be described in detail. FIG. 8 is a perspective view schematically showing a structure of the lens 88. FIG. 9A is a side sectional view of the lens 88. FIG. 9B is a side sectional view showing a part of the reflected light lens 88b in enlarged dimension.

As described above, the lens 88 is formed by integrally molding the irradiation light lens 88a for condensing the irradiation light and the reflected light lens 88b for condensing the reflected light. The lens 88 can be molded by using a glass material, a resin material, or the like. The lens 88 includes a retained portion 140 to be mounted and retained by the mounting portion 87 of the photometric block 72, an irradiation light convex portion 142 having a function of the irradiation light lens 88a, and a reflected light convex portion 144 having a function of the reflected light lens 88b.

As shown in FIG. 9A, in the lens 88, the irradiation light convex portion 142 and the reflected light convex portion 144 are formed on one surface of the retained portion 140, and another surface of the retained portion 140 is formed in a flat shape. That is, the irradiation light lens 88a and the reflected light lens 88b share the retained portion 140. In this case, the irradiation light lens 88a functions as a planoconvex lens of the irradiation light convex portion 142 and the retained portion 140. The reflected light lens 88b similarly functions as a planoconvex lens of the reflected light convex portion 144 and the retained portion 140.

As shown in FIG. 8, the retained portion 140 is formed in an oblong circular shape as a whole, and has a groove portion 146 formed in a central portion of a side circumferential surface of the retained portion 140. The groove portion 146 is carved along a circumferential direction of the side circumferential surface, and is fitted with the O-ring 90 when the lens 88 is inserted into the mounting portion 87.

In addition, as shown in FIG. 9A, the irradiation light convex portion 142 is formed as a spherical lens on a surface of the retained portion 140. In this case, the radius of curvature of the spherical surface of the spherical lens is adjusted on the basis of intervals between the test paper 70, the lens 88, and the light emitting elements 100. In the blood glucose level measuring device 10 according to the present embodiment, the spherical lens is formed such that the spherical surface of the irradiation light convex portion 142 is disposed at substantially an intermediate position between the light emitting elements 100 and the test paper 70 (see FIG. 6), so that the shape of the spherical lens can be designed relatively easily.

The reflected light convex portion 144 includes a cylindrical portion (projecting portion) 148 projecting from the surface of the retained portion 140 by a predetermined amount and a condensing portion 150 formed as an aspheric lens on the cylindrical portion 148. The cylindrical portion 148 is formed with such an outside diameter as to be engaged with a communicating portion 110a (see FIG. 6) of the reflected light optical path 110 which communicating portion 110a is formed at a position of connection to the mounting portion 87 of the photometric block 72, and is fitted into the communicating portion 110a of the reflected light optical path 110 by the mounting of the lens 88. Thereby, the condensing portion 150 provided on the cylindrical portion 148 can be surely positioned at a predetermined position in the reflected light optical path 110.

The surface of the condensing portion 150 is formed as that of an aspheric lens. An aspheric lens is a lens formed in a curved surface that does not constitute a spherical surface, to which lens a hyperboloid or a high-order polynomial curved surface, for example, corresponds. Because the condensing portion 150 is formed as an aspheric lens, the condensing portion 150 condenses the reflected light reflected from the test paper 70 onto the surface of the light receiving element 102 so as not to cause spherical aberration.

Describing the condensing portion 150 (aspheric lens) according to the present embodiment more concretely, as shown in FIG. 9B, the curved surface (solid line in FIG. 9B) of the aspheric lens has a central portion thereof formed at a same position as that of the curved surface of an ordinary spherical lens (dotted line in FIG. 9B), and is formed so as to be a curved surface whose slope, from the central portion toward a peripheral portion, is steep at first as compared with the curved surface of the spherical lens, and then becomes gradually gentle. Incidentally, while FIG. 9 shows a greatly recessed state of the peripheral portion of the reflected light lens 88b, the curved surface of an actual aspheric lens is formed with a very slight difference in shape (for example a few µm) from the spherical lens. The curved surface of the aspheric lens is designed appropriately on the basis of the index of refraction and shape (diameter, thickness, and radius of curvature) of the lens 88, arrangement intervals between the reflected light lens 88b, the light receiving element 102, and the test paper 70, or the like.

In addition, the lens 88 is formed such that an optical axis La of the irradiation light lens 88a (irradiation light convex portion 142) and an optical axis Lb of the reflected light lens 88b (reflected light convex portion 144) are substantially parallel to each other. This facilitates the processing of the lens 88 as compared with a constitution in which the optical axes La and Lb of the irradiation light lens 88a and the reflected light lens 88b are inclined with respect to each other, for example, when the irradiation light lens 88a and the reflected light lens 88b are molded integrally with each other. In the case in which the reflected light convex portion 144 is formed as an aspheric lens, in particular, advanced processing techniques are required. Thus, it is also possible to improve processing efficiency by making the optical axes La and Lb parallel to each other.

When the lens 88 is mounted in the photometric block 72, the O-ring 90 is mounted in the groove portion 146 of the retained portion 140, and thereafter the lens 88 is inserted into the mounting portion 87 from the projecting portion side opening portion 86. The projecting portion side opening portion 86 of the photometric block 72 is formed in such a shape as to be engaged with the plane shape (oblong circular shape) of the lens 88, and further the mounting portion 87 is formed by cutting the plane shape to a predetermined depth. Therefore, at a time of mounting the lens 88, the lens 88 can be easily guided into the mounting portion 87 by inserting the lens 88 from the direction of the opening of the projecting portion side opening portion 86. That is, according to the photometric block 72, excellent mountability can be exerted in the mounting of the lens 88.

As shown in FIG. 6, in a state in which the lens 88 is mounted and retained by the mounting portion 87, the wall portion 109 within the photometric block 72 abuts against the retained portion 140, so that a space in which the irradiation light convex portion 142 is disposed (irradiation light optical path 108) and a space in which the reflected light convex portion 144 is disposed (reflected light optical path 110) are isolated from each other. In addition, an opening end portion of the mounting portion 87 (distal end of the projecting portion side opening portion 86) is swaged to the inside by heat swaging or the like. Thus swaging the opening end portion of the projecting portion side opening portion 86 prevents the lens 88 from falling off, and allows the mounting portion 87 to retain the lens 88 more securely.

In addition, in the state in which the lens 88 is mounted and retained by the mounting portion 87, the irradiation light convex portion 142 is opposed to the light emitting elements 100, and the reflected light convex portion 144 is opposed to the light receiving element 102. As described above, the cylindrical portion 148 of the reflected light convex portion 144 is fitted into the communicating portion 110a. In a constitution without the cylindrical portion 148, for example, the condensing portion formed in the aspheric lens may be displaced, which decreases the reflected light condensing accuracy of the condensing portion. On the other hand, the cylindrical portion 148 in the reflected light convex portion 144 according to the present embodiment is fitted so that the condensing portion 150 can be positioned securely. Hence, the reflected light lens 88b can stably condense the reflected light on the light receiving element 102.

In addition, the proximal end portion 76 and the projecting portion 78 of the photometric block 72 are closed in a circumferential direction, so that light entering the reflected light optical path 110 other than the reflected light can be reduced. That is, the photometric block 72 is formed in a shape in which a sufficient light shielding property within the reflected light optical path 110 is ensured.

The blood glucose level measuring device 10 according to the present embodiment is basically formed as described above. The measurement of a blood component by the blood glucose level measuring device 10 will next be described. In the measurement of a blood component, the blood of a measured person is first collected by using the blood glucose level measuring device 10 mounted with the measuring tip 58. Specifically, a fingertip of the measured person is punctured by using a dedicated puncture device (not shown) to make a small amount (about 0.3 to 1.5 µL, for example) of blood flow out onto a skin surface. Then, the distal end of the nozzle 62 of the measuring tip 58 mounted on the distal end of the blood glucose level measuring device 10 is made to abut on the blood flowed onto the fingertip.

Thereby, the blood enters the inside of the collecting hole 62a through the recessed groove 62b of the distal end of the nozzle 62, and is sucked to the rear end due to capillarity. Then, the blood infiltrates into the test paper 70 housed within the test paper housing portion 68, and spreads in a circular shape toward the outside in the radial direction of the test paper 70. At the same time as the spreading of the blood, reaction between the glucose in the blood and a coloring reagent included in the test paper 70 is started, so that the test paper 70 is colored depending on the amount of the glucose.

As shown in FIG. 7, when the blood glucose level measuring device 10 optically detects the coloration of the test paper 70, the irradiation light Li is first emitted from the first light emitting element 100a (or the second light emitting element 100b). The irradiation light Li emitted from the first light emitting element 100a (or the second light emitting element 100b) passes through the irradiation light optical path 108, and enters the irradiation light lens 88a (irradiation light convex portion 142). When the irradiation light Li enters the irradiation light convex portion 142, the irradiation light convex portion 142 refracts the light rays of the irradiation light Li in the direction of the optical axis La according to the curved surface of the irradiation light convex portion 142. The light rays of the irradiation light Li refracted by the irradiation light convex portion 142 pass through the inside of the lens as they are, and are emitted from the retained portion 140 and condensed at a focal position A of the test paper 70.

When the irradiation light Li applied to the test paper 70 is applied at the focal position A of the test paper 70, the irradiation light Li is divided into direct reflected light reflected in a direction of incidence of the irradiation light Li and scattered light scattered to the periphery of the direct reflected light. As shown in FIG. 6, the reflected light lens 88b is situated at a position shifted by a predetermined angle (for example 30°) with respect to the focal position A of the test paper 70, and the scattered light enters the reflected light lens 88b as reflected light Lr. In addition, the light receiving element 102 for receiving the reflected light Lr is disposed at a position symmetric to the focal position A of the test paper 70 with respect to a point of intersection B where the optical axis Lb of the reflected light convex portion 144 and the vertex portion of the reflected light convex portion 144 intersect each other. The reflected light lens 88b emits the reflected light Lr such that the reflected light Lr is condensed at the position of disposition of the light receiving element 102.

FIG. 10 is a schematic diagram of assistance in explaining the travel of the light rays Lr0 to Lr2 of the reflected light within the reflected light lens 88b. When the light rays Lr0 to Lr2 of the reflected light reflected from the test paper 70 are made incident on the reflected light lens 88b, the light rays Lr0 to Lr2 are refracted by a predetermined angle depending on a difference between the index of refraction of a space (air) and the index of refraction of the lens 88. Within the retained portion 140 and the cylindrical portion 148 of the reflected light lens 88b, the light rays Lr0 to Lr2 of the reflected light refracted by the predetermined angle travel in straight lines as they are, and are guided to the condensing portion 150. The condensing portion 150 refracts the light rays Lr0 to Lr2 of the reflected light according to the curved surface of the aspheric lens.

Describing the travel of the light rays Lr0 to Lr2 of the reflected light within the reflected light lens 88b concretely, when the light ray Lr0 of the reflected light which light ray passes through the vertex portion of the reflected light convex portion 144 shown in FIG. 10 enters at an angle θ with the optical axis Lb of the reflected light convex portion 144, the light ray Lr0 of the reflected light is emitted at an angle -θ from the vertex portion of the reflected light convex portion 144. Then, the light ray Lr0 of the reflected light travels in a straight line as it is within the reflected light optical path 110, and enters the surface (focal position C) of the opposed light receiving element 102 at an angle ϕ (ϕ = 90° - θ) with the surface of the light receiving element 102.

In addition, the light ray Lr1 of the reflected light which light ray passes through an upper portion of the reflected light convex portion 144 in FIG. 10 slightly inclines an angle of emission thereof toward the optical axis Lb due to the gently curved surface of the condensing portion 150 (aspheric lens). As a result, the light ray Lr1 of the reflected light is emitted in a state of being slightly refracted to the inside from the upper portion of the reflected light convex portion 144, and is focused on the light ray Lr0 at the surface (focal position C) of the light receiving element 102.

Further, the light ray Lr2 of the reflected light which light ray passes through a lower portion of the reflected light convex portion 144 in FIG. 10 enters the gently curved surface of the condensing portion 150 (aspheric lens) at a larger angle than the light ray Lr1, and inclines an angle of emission thereof toward the optical axis Lb more than the light ray Lr1. As a result, the light ray Lr1 of the reflected light is emitted in a state of being refracted to the inside from the lower portion of the reflected light convex portion 144, and is focused on the light ray Lr0 at the surface (focal position C) of the light receiving element 102.

The reflected light lens 88b thus condenses the light rays Lr0 to Lr2 of the reflected light at the predetermined focal position C on the surface of the light receiving element 102. In general, when the condensing portion 150 is a spherical lens, the focal positions C of the light rays Lr0 to Lr2 of the reflected light are shifted from each other in the direction of the optical axis Lb. On the other hand, in the present embodiment, because the condensing portion 150 is formed as an aspheric lens, the focal positions C of the light rays Lr1 and Lr2 of the reflected light which light rays are passed through the peripheral portion of the reflected light convex portion 144 and the light ray Lr0 of the reflected light which light ray is passed through the vertex portion of the reflected light convex portion 144 coincide with each other, so that spherical aberration can be reduced greatly.

When the light receiving element 102 receives the reflected light Lr, the light receiving element 102 detects the light amount of the reflected light Lr. The blood glucose level measuring device 10 measures the degree of coloration of the test paper 70 on the basis of the detected value.

In the measurement of a blood glucose level by the blood glucose level measuring device 10, the irradiation light Li of the first light emitting element 100a and the irradiation light Li of the second light emitting element 100b are emitted alternately. Then, coloring material produced by the reaction between the coloring reagent and the glucose is detected with the irradiation light Li applied by the first light emitting element 100a, and coloration density corresponding to the amount of the glucose is measured. In addition, red blood cells are detected with the irradiation light Li applied by the second light emitting element 100b, and the red-color density of the red blood cells is measured. Then, a blood glucose level can be obtained by correcting a glucose value obtained from the coloration density using a hematocrit value obtained from the red-color density.

When the measuring tip 58 is removed from the casing 12 after the measurement, the ejecting member 48 is slid to the front (distal end) by pressing the ejecting operating element 44 to the distal end. The pushing-out portion 116 of the ejecting member 48 thereby presses the locking pawls 66 of the measuring tip 58 to the front, so that the measuring tip 58 can be removed.

In this case, the user can easily remove the measuring tip 58 from the blood glucose level measuring device 10 by one-hand operation. In addition, because the measuring tip 58 is attached to the distal end of the casing 12 bend to the lower case 18 side, the measuring tip 58 can be disposed of easily and quickly by operating the ejecting operating element 44 without hands touching the measuring tip 58.

FIG. 11 is a schematic diagram of assistance in explaining a modification of the lens according to the present invention. As shown in FIG. 11, a reflected light lens 162 of a lens 160 may be formed such that a condensing portion 166 of a reflected light convex portion 164 is formed as a spherical lens and an aspheric lens 168 molded from a resin material is laminated to the surface of the spherical lens. In this case, the aspheric lens 168 is molded with the shape (diameter and radius of curvature) of the condensing portion 166 also taken into consideration so that the surface of the aspheric lens 168 is the same curved surface as the curved surface of the condensing portion 150 (aspheric lens) in FIG. 9. Advanced techniques are generally required for the processing of aspheric lenses. Therefore, a constitution in which the aspheric lens 168 is thus molded separately from the processing of the lens 160 and the aspheric lens 168 is laminated can be manufactured at lower cost than in the case where the aspheric lens is directly processed in the lens 88.

In addition, when the reflected light Lr is made obliquely incident on the reflected light lens 88b, refraction occurs within the lens on the basis of Snell's law. Thus, the angle of incidence θ of the light ray Lr0 of the reflected light can be changed by adjusting the plate thickness of the retained portion 140. That is, when an optimum angle of incidence θ of the light ray Lr0 of the reflected light is determined from relation between the arrangement positions of the test paper 70, the lens 88 (reflected light lens 88b), and the light receiving element 102, and the plate thickness of the retained portion 140 is set such that the optimum angle of incidence θ is obtained, the reflected light lens 88b can be easily made to condense the reflected light Lr reflected from the test paper 70 onto the predetermined focal position C on the light receiving element 102. Therefore, after the condensing portion 150 is processed into an aspheric lens, the focal position of the reflected light can be corrected by adjusting the plate thickness of the retained portion 140 without changing the shape of the aspheric lens.

As described above, the blood glucose level measuring device 10 is provided with the mounting portion 87 conforming to the plane shape of the lens 88 in the photometric block 72, whereby the mountability of the lens 88 can be improved and the efficiency of device assembly can be increased. In addition, because the reflected light lens 88b is formed as an aspheric lens, the spherical aberration of the reflected light Lr can be reduced greatly, so that the accuracy of measurement of a biological component can be improved.

It is to be noted that the present invention is not limited to the foregoing embodiments, but various constitutions can of course be adopted without departing from the spirit of the present invention. For example, the component measuring device according to the present invention may be adopted as a device for measuring a component of urine, or can be adopted as a device for measuring a component of drainage water, industrial water, or the like other than a body fluid.

In addition, the shape of the reflected light convex portion 144 does not need to be formed only for the reduction of spherical aberration. That is, the reflected light convex portion 144 can condense the reflected light Lr with higher accuracy by being formed with an optimum curved surface with comatic aberration, astigmatism, field curvature, distortion aberration, axial chromatic aberration, chromatic aberration of magnification, and the like known as aberrations taken into consideration.

## Claims

1. A component measuring device (10) comprising a light emitting element (100) adapted to irradiate a measurement object (70) with irradiation light for measurement, a light receiving element (102) adapted to detect reflected light reflected from the measurement object (70), and a measuring portion adapted to measure a component in a fluid impregnated into the measurement object (70) on a basis of a detected value of the reflected light, the component measuring device (10) comprising:
a block body (72) having a mounting portion (87) having an opening so as to be opposed to the measurement object (70), and having a reflected light optical path (110) adapted to guide the reflected light to the light receiving element (102), the reflected light optical path (110) communicating with the mounting portion (87); and
a reflected light lens (88b) adapted to condense the reflected light onto the light receiving element (102),
wherein the reflected light lens (88b) is formed as an aspheric lens for reducing at least spherical aberration of the reflected light on a basis of a position of disposition of the light receiving element (102), and is disposed on the reflected light optical path (110) by being inserted into the mounting portion (87) from the front side of the opening of the mounting portion (87),
the block body (72) has an irradiation light optical path (108) adapted to guide the irradiation light to the measurement object (70),
an irradiation light lens (88a) adapted to condense the irradiation light onto the measurement object (70) is disposed on the irradiation light optical path (108), and
the reflected light lens (88b) and the irradiation light lens (88a) are arranged so as to share a retained portion (140) retained by the mounting portion (87), and molded integrally with each other,
**characterized in that**
an optical axis of the irradiation light lens (88a) and an optical axis of the reflected light lens (88b) are formed substantially in parallel with each other, and
the aspheric lens is formed in a shape reducing the spherical aberration of the reflected light incident at a predetermined angle with the optical axis of the reflected light lens (88b).

2. The component measuring device (10) according to claim 1, **characterized in that**
the reflected light lens (88b) includes
a projecting portion (148) projecting by a predetermined amount from the retained portion (140) toward the reflected light optical path (110), and
a condensing portion (150) formed as the aspheric lens on the projecting portion (148).

3. The component measuring device (10) according to claim 1, **characterized in that**
an opening end portion of the mounting portion (87) is swaged to an inside in a state of the reflected light lens (88b) being inserted within the mounting portion (87).

4. The component measuring device (10) according to claim 1, **characterized in that**
the component measuring device (10) is a blood glucose level measuring device for measuring mainly a blood glucose level of blood components in blood as body fluid.

## Patentansprüche

1. Bestandteilmessgerät (10), mit einem Lichtemissionselement (100), das angepasst ist, zum Messen ein Messobjekt (70) mit Bestrahlungslicht zu bestrahlen, einem Lichtempfängerelement (102), das angepasst ist, reflektiertes Licht zu erfassen, das von dem Messobjekt (70) reflektiert wird, und einem Messabschnitt, der angepasst ist, auf einer Basis eines erfassten Werts des reflektierten Lichts einen Bestandteil in einem Fluid zu messen, mit dem das Messobjekt (70) durchtränkt ist, wobei das Bestandteilmessgerät (10)
einen Blockkörper (72) mit einem Montageabschnitt (87), der eine Öffnung hat, um dem Messobjekt (70) gegenüberzuliegen, und einen optischen Pfad (110) für reflektiertes Licht hat, der angepasst ist, das reflektierte Licht zu dem Lichtempfängerelement (102) zu führen, wobei der optische Pfad (110) für reflektiertes Licht mit dem Montageabschnitt (87) in Verbindung steht; und
eine Linse (88b) für reflektiertes Licht hat, die angepasst ist, das reflektierte Licht auf dem Lichtempfängerelement (102) zu sammeln, wobei
die Linse (88b) für reflektiertes Licht als eine asphärische Linse zum Verringern von zumindest einer sphärischen Aberration des reflektierten Lichts auf einer Basis einer Anordnungsposition des Lichtempfängerelements (102) ausgebildet ist und auf dem optischen Pfad (110) für reflektiertes Licht angeordnet ist, indem sie von der Vorderseite der Öffnung des Montageabschnitts (87) in den Montageabschnitt (87) eingesetzt ist,
der Blockkörper (72) einen optischen Pfad (108) für Bestrahlungslicht hat, der angepasst ist, das Bestrahlungslicht zu dem Messobjekt (70) zu führen,
eine Linse (88a) für Bestrahlungslicht, die angepasst ist, das Bestrahlungslicht auf dem Messobjekt (70) zu sammeln, auf dem optischen Pfad (108) für Bestrahlungslicht angeordnet ist und
die Linse (88b) für reflektiertes Licht und die Linse (88a) für Bestrahlungslicht angeordnet sind, um einen gehaltenen Abschnitt (140) zu teilen, der durch den Montageabschnitt (87) gehalten ist, und einstückig miteinander ausgeformt sind, **dadurch gekennzeichnet, dass**
eine optische Achse der Linse (88a) für Bestrahlungslicht und eine optische Achse der Linse (88b) für reflektiertes Licht im Wesentlichen parallel zueinander ausgebildet sind und
die asphärische Linse in einer Form ausgebildet ist, die die sphärische Aberration des reflektierten Lichts verringert, das in einem vorbestimmten Winkel zu der optischen Achse der Linse (88b) für reflektiertes Licht einfällt.

2. Bestandteilmessgerät (10) nach Anspruch 1, **dadurch gekennzeichnet, dass**
die Linse (88b) für reflektiertes Licht
einen Vorsprungsabschnitt (148), der durch einen vorbestimmten Betrag von dem gehaltenen Abschnitt (140) in Richtung des optischen Pfads (110) für reflektiertes Licht vorsteht und
einen Sammelabschnitt (150) hat, der als die asphärische Linse an dem Vorsprungsabschnitt (148) ausgebildet ist.

3. Bestandteilmessgerät (10) nach Anspruch 1, **dadurch gekennzeichnet, dass**
ein Öffnungsendabschnitt des Montageabschnitts (87) in einem Zustand, in dem die Linse (88b) für reflektiertes Licht in den Montageabschnitt (87) eingesetzt ist, zu einer Innenseite gestaucht ist.

4. Bestandteilmessgerät (10) nach Anspruch 1, **dadurch gekennzeichnet, dass**
das Bestandteilmessgerät (10) ein Blutzuckerspiegelmessgerät zum Messen von hauptsächlich einem Blutzuckerspiegel von Blutbestandteilen in Blut als einer Körperflüssigkeit ist.

## Revendications

1. Dispositif de mesure de composant (10) comprenant un élément d'émission de lumière (100) conçu pour irradier un objet de mesure (70) avec une lumière d'irradiation pour la mesure, un élément de réception de lumière (102) conçu pour détecter une lumière réfléchie qui est réfléchie par l'objet de mesure (70) et une partie de mesure conçue pour mesurer un composant dans un fluide imprégné dans l'objet de mesure (70) sur la base d'une valeur détectée de la lumière réfléchie, le dispositif de mesure de composant (10) comprenant :
un corps de bloc (72) possédant une partie de montage (87) ayant une ouverture de manière à être opposée à l'objet de mesure (70) et possédant un trajet optique de lumière réfléchie (110) conçu pour guider la lumière réfléchie vers l'élément de réception de lumière (102), le trajet optique de lumière réfléchie (110) communiquant avec la partie de montage (87) ; et
une lentille de lumière réfléchie (88b) conçue pour condenser la lumière réfléchie sur l'élément de réception de lumière (102),
dans lequel la lentille de lumière réfléchie (88b) est sous la forme d'une lentille asphérique pour réduire au moins l'aberration sphérique de la lumière réfléchie sur la base d'une position de disposition de l'élément de réception de lumière (102), et est disposée sur le trajet optique de lumière réfléchie (110) en étant insérée dans la partie de montage (87) à partir du côté avant de l'ouverture de la partie de montage (87),
le corps de bloc (72) possède un trajet optique de lumière d'irradiation (108) conçu pour guider la lumière d'irradiation vers l'objet de mesure (70),
une lentille de lumière d'irradiation (88a) conçue pour condenser la lumière d'irradiation sur l'objet de mesure (70) est disposée sur le trajet optique de lumière d'irradiation (108), et
la lentille de lumière réfléchie (88b) et la lentille de lumière d'irradiation (88a) sont agencées de manière à partager une partie retenue (140) qui est retenue par la partie de montage (87) et moulées de manière solidaire l'une avec l'autre,
**caractérisé en ce que**
un axe optique de la lentille de lumière d'irradiation (88a) et un axe optique de la lentille de lumière réfléchie (88b) sont formés sensiblement en parallèle l'un à l'autre, et
la lentille asphérique présente une forme réduisant l'aberration sphérique de la lumière réfléchie incidente à un angle prédéterminé avec l'axe optique de la lentille de lumière réfléchie (88b).

2. Dispositif de mesure de composant (10) selon la revendication 1, **caractérisé en ce que** la lentille de lumière réfléchie (88b) inclut
une partie en saillie (148) faisant saillie d'une quantité prédéterminée par rapport à la partie retenue (140) vers le trajet optique de lumière réfléchie (110), et
une partie de condensation (150) formée par la lentille asphérique sur la partie en saillie (148).

3. Dispositif de mesure de composant (10) selon la revendication 1, **caractérisé en ce que**
une partie d'extrémité d'ouverture de la partie de montage (87) est emboutie dans un intérieur dans un état dans lequel la lentille de lumière réfléchie (88b) est insérée à l'intérieur de la partie de montage (87).

4. Dispositif de mesure de composant (10) selon la revendication 1, **caractérisé en ce que**
le dispositif de mesure de composant (10) est un dispositif de mesure de taux de glycémie pour mesurer principalement un taux de glycémie de composants sanguins dans le sang en tant que fluide corporel.
